# EUROPEAN PATENT SPECIFICATION

(11) **EP 0 930 898 B1**
(45) Date of publication and mention of the grant of the patent: **20.02.2002**
(21) Application number: 97939038.2
(22) Date of filing: 05.09.1997
(51) Int. Cl.: A61L 15/28, A61L 26/00

(54) **WOUND DRESSINGS**
WUNDVERBÄNDE
PANSEMENTS

(30) Priority: 05.09.1996 GB 9618570
(43) Date of publication of application: 28.07.1999
(73) Proprietor: Acordis Speciality Fibres Limited, Spondon, Derby DE21 7BP (GB)
(72) Inventor: THOMPSON, Joseph, Coventry CV3 5BW (GB); PATEL, Champa, Coventry CV6 5JY (GB)
(74) Representative: Hale, Stephen Geoffrey
(86) International application number: GB9702384
(87) International publication number: WO9809663

(56) References cited:
- WO-A-93/12275
- WO-A-94/16746
- GB-A- 570 428
- GB-A- 2 060 018
- GB-A- 2 120 696
- GB-A- 2 276 819
- US-A- 4 579 943

## Description

This invention relates to viscous pasty materials suitable for use as wound-contacting layers in wound dressings and to processes for their manufacture.

### Background art

EP-A-0,586,260 and US-A-5,482,932 describe an alginate gel containing from 2 to 11 percent by weight alginate (expressed as alginic acid), which gel takes the form of a viscous paste exhibiting visible fibrous structure. Such gels can be made by repeatedly subjecting a loose assembly of water-insoluble or water-swellable alginate fibres, for example alginate fibres containing an insolubilising cation such as calcium, to a cycle of immersion in an aqueous liquor containing a solubilising cation for alginate, for example a liquor containing sodium ions such as a saline solution, and expression of excess liquor, until the gel has formed. A single immersion and expression cycle generally results in the production of a gelatinised fibrous mass rather than the deformable paste ultimately desired, or alternatively it permits the manufacture only of products of very high salt content. EP-A-0,586,260 and US-A-5,482,932 also state that such gels can be made in a single step by mixing a suitable water-insoluble or water-swellable alginate fibre with a solution containing a solubilising cation without subsequent expression, but they point out that in this method it may be difficult to balance the need for sufficient mixing to produce a homogeneous gel against the need to avoid excessive mixing which would destroy the desired fibrous structure. EP-A-0,586,260 and US-A-5,482,932 further state that such gels can also be made by treatment of water-soluble alginate fibres, for example sodium alginate fibres, with an aqueous solution containing an insolubilising cation, for example calcium.

Although alginate gels of this type have value as wound dressings, they and their method of production exhibit a number of disadvantages. Repeated immersion of calcium alginate fibre in aqueous liquor followed by expression of excess liquor can result in appreciable losses (up to about 20 percent) of the alginate, dissolved in the expressed liquor. This is undesirable on both commercial and economic grounds. These alginate gels have a greenish tint and a characteristic odour, which may be found aesthetically undesirable in medical applications. These alginate gels tend to exhibit syneresis after sterilisation by autoclaving, whereby the gel undesirably tends to separate into gel and liquid fractions. Furthermore, the degree of syneresis tends to increase with increased time and temperature in autoclaving, making difficult the production of gels with uniform properties and appearance, particularly on a large scale. It is an object of the present invention to provide a gel useful for medical purposes and a method of making the same which overcome such disadvantages.

### Disclosure of the invention

According to a first aspect of the invention, there is provided a method for the manufacture of a material suitable for use as a wound dressing, characterised in that from 2 to 10 parts by weight of polymer solids, said polymer solids consisting of at least 50 percent by weight of water-swellable cellulose ether fibre and no more than 50 percent by weight of a powder which is a water-swellable or water-soluble cellulose ether or other water-swellable or water-soluble polymer, and correspondingly from 98 to 90 parts by weight of an aqueous liquor are mixed under low-shear conditions, thereby forming said material in the form of a viscous paste exhibiting visible fibrous structure. For example, the polymer solids may consist to the extent of at least 75 percent by weight of the fibre or they may consist only of the fibre. The fibre and powder may be of the same or different cellulose ethers. Examples of water-swellable or water soluble polymers other than cellulose ethers include known polymers based on acrylic acid.

According to a second aspect of the invention, there is provided a wound dressing which comprises a viscous paste exhibiting visible fibrous structure, characterised in that the paste consists of from 2 to 10 percent by weight of polymer, at least 50 percent by weight of said polymer being a water-swellable polymer and the balance (if any) of said polymer being a water-soluble polymer, and at least 50 percent by weight of said polymer being a cellulose ether, and correspondingly of from 98 to 90 percent by weight of an aqueous liquor. For example, the polymer may consist to the extent of at least 75 percent by weight of cellulose ether or it may consist only of cellulose ether. This viscous paste is readily deformable, for example by moulding, spreading or extrusion through an orifice. It has the general appearance of a transparent or translucent hydrocolloid or hydrogel. Fibrous structure can be seen in the paste (gel) of the invention either with the naked eye or microscopically, and fibrous material can be manually drawn in small amounts from the paste; these features are essential characteristics of the wound dressings of the invention.

Suitable cellulose ethers include neutral cellulose ethers such as methylcellulose, ethylcellulose and hydroxypropylmethylcellulose. Ionic cellulose ethers, in particular anionic cellulose ethers such as carboxymethylcellulose (CMC), may be preferred. Mixed ethers and mixtures of cellulose ethers may be used. The manufacture of fibres suitable for use in the invention is described in WO-A-93/12275. The cellulose ether may if desired be crosslinked. Both water-swellable and water-soluble cellulose ethers are known.

The titre of the fibres used in the method of the invention may be in the range from 0.5 to 10 decitex. The fibres used in the method of the invention are preferably staple fibres having a length in the range from 1 to 50, often from 5 to 50, mm. Very short fibres tend to yield products with little or none of the desired fibrous character. Very long fibres tend to yield products of "stringy" appearance, which are difficult to package and to extrude.

The cellulose ether fibre used in the method of the invention preferably exhibits liquor retention (saline solution), measured as described in WO-A-93/12275, Example 11, at a pressure of 3.42 kPa, in the range from 15 to 35 g/g.

The degree of substitution (D.S.) of the cellulose ether used in the invention is preferably in the range from 0.1 to 1.0, more preferably 0.2 to 0.5.

The aqueous liquor may be water, but it is more often a saline solution containing from 0.1 to 5.0, often about 0.9, percent by weight sodium chloride, particularly physiological saline. If desired, the aqueous liquor may contain ions which exhibit physiological activity, for example silver or zinc cations. The aqueous liquor may additionally comprise one or more other dissolved or dispersed substances which it is desired to incorporate into the dressing. Examples of such substances include conventional substances used as preservatives, humectants such as propylene glycol, antimicrobial agents, pharmaceutical agents, analgesics, non-stick compositions such as silicone emulsions, wound-healing agents, odour-absorbing agents and fragrances. An aqueous liquor which comprises from 5 to 30 or 5 to 25 percent by weight propylene glycol may be used.

The method of the invention may conveniently be performed at ambient temperature.

The method of the invention has the advantages that it enables precise control of the proportions and amounts of all substances present in the product of the method and that it involves a minimum of losses or waste.

The intensity and extent of mixing used in the method of the invention should be sufficient to ensure good mixing and the production of a homogeneous product but should not be so great that the essential fibrous structure of the product is destroyed. For example, the components may be mixed together using a paddle or such like mixer which achieves mixing under low shear conditions.

The method of the invention may be carried out continuously, but it may be found more convenient to carry it out batchwise. Conveniently, it may be performed by adding the fibres to the aqueous liquor with a sufficient degree of stirring to ensure wetting out and uniform mixing. If a product containing propylene glycol is to be prepared, it may be found advantageous to add the powder (if any) and the fibres with stirring to the propylene glycol, the propylene glycol optionally being in admixture with some water or saline solution, in order to disperse the powder (if any) and the fibres without appreciable swelling. The method may then be completed by adding the remainder of the aqueous liquor to the resulting dispersion so that the desired product is formed.

If desired, a small proportion of non-swellable fibre, for example viscose rayon fibre, may be included in the pastes of the invention as reinforcement; although such is generally not required.

Liquor handling (absorption and donation) properties (capacity and rate) are important properties of wound dressing materials such as those of the invention. The absorption properties of the gels disclosed in EP-A-0,586,260 and US-A-5,482,932 are primarily determined by the degree of expression (dewatering) employed in the final step of manufacture, which controls the proportion of alginate in the gel. Absorption capacity increases with the proportion of alginate in the gel. Other factors such as the nature and ratio of the cations in the alginate gel influence absorption properties to a lesser extent. The maximum level of absorption capacity is therefore influenced by the practical difficulties of pressing liquor from the gel in this final step, both as regards the mechanical process of expression itself and the desire to obtain a homogeneous pasty product. In contrast, the method of the invention involves only a single gel-forming step and readily permits the manufacture of gels (pastes) of relatively high polymer content. The upper limit on polymer content is largely determined by the need for the presence of sufficient liquor to wet the cellulose ether fibres thoroughly and uniformly when first mixed.

The wound dressings of the invention are colourless and free from odour. They can be sterilised by conventional autoclaving techniques without material change to their appearance or physical and chemical properties. In comparison with gels made from water-swellable polymer in powder form alone, the pastes of the invention exhibit advantageously low stickiness. They are therefore convenient to handle. They further exhibit a high rate of absorption and high permeability to liquids. They further retain good integrity (i.e. do not flow excessively) after having absorbed liquid. It is thought that the pastes of the invention may be considered as consisting of an assembly of smaller gels and that the aforementioned properties arise from this phenomenon.

The invention is illustrated by the following Example, in which parts and proportions are by weight unless otherwise specified:-

### Example 1

Carboxymethylated lyocell fibre (available from Courtaulds Fibres (Holdings) Limited) (1.7 dtex) was cut to staple length (15 or 50 mm) and carded by hand or machine in order to open any clumps of fibre. The fibre was placed in a glass beaker and to it was added the requisite quantity of saline solution (0.9% NaCl) to give total weight 100 g with fibre contents 5%, 6% or 7%. The mixture was stirred by hand for about 5 minutes until the fibre was thoroughly wetted and a homogeneous product had been obtained. The product was sterilised in an autoclave at 121°C for 25 minutes. Product appearance was not changed in any essential respect by autoclaving. The product had the appearance of a viscous pasty mass which could be moulded, and it exhibited visible fibrous structure. Fibrous material could be withdrawn from the product with the aid of tweezers.

The liquor retention of the fibres used was measured by the technique described in WO-A-93/12275 in Example 11, using a pressure of 3.42 kPa. It has been found that liquor retention over the range from 15 to 35 g/g corresponds roughly to D.S. over the range from 0.2 to 0.4. Absorption capacity and stiffness of the products of the method of the invention were measured using the techniques described in US-A-5,482,932 in Examples 12 and 13 respectively.

The ability of a wound dressing to donate moisture is important where rehydration and autolysis of dead tissue (debridement) are required. Moisture donation properties were measured as follows. A 60 ml plastics syringe of internal diameter 28 mm is taken and the delivery end cut off at the first graduation mark. The plunger is withdrawn to the 30 ml mark, and the modified syringe is placed mouth upwards on a level surface. A freshly-prepared gelatin solution (10 g) at 60°C is poured into the syringe and allowed to cool and set. The gelatin solution was prepared as follows. To 65 ± 0.02 g of sodium chloride and calcium chloride solution BP in a wide-mouth container is added sufficient gelatin powder (175 Bloom) to produce a total weight of 100 ± 0.02 g. The container is sealed, shaken vigorously for at least 20 sec, then held at a temperature of 60°C overnight to form a clear homogeneous solution.

The material to be tested (10 g) is placed on the gelatin and the syringe is sealed with a foil cap and adhesive tape. The assembly is then stored at 25°C for 48 hours. The material under test is reweighed and its change in weight recorded.

The results shown in Tables 1, 2 and 3 were obtained:-

**Table 1 -**

| **Absorption Capacity, g/g** | | | |
|---|---|---|---|
| **Liquor Retention** **g/g** | **5% fibre/15 mm** | **6% fibre/15 mm** | **7% fibre/15 mm** |
| 16 | 0.30 | - | - |
| 23 | 0.30 | 0.50 | - |
| 27* | - | - | 0.70 |
| 27 | - | - | 0.70 |

**Table 2 -**

| **Stiffness, cN** | | | | |
|---|---|---|---|---|
| **Liquor Retention** **g/g** | **5%** **fibre/15mm** | **5%** **fibre/50mm** | **6%** **fibre/15mm** | **7%** **fibre/15mm** |
| 16 | 99 | 46 | - | - |
| 23* | - | 93 | - | - |
| 23 | 114 | 130 | 264 | 352 |
| 31 | 164 | 133 | 285 | - |

**Table 3 -**

| **Moisture Donation, g/g** | | |
|---|---|---|
| **Liquor Retention g/g** | **5% fibre/15 mm** | **6% fibre/15 mm** |
| 16 | 0.15 | - |
| 23 | 0.18 | 0.14 |
| 31 | 0.15 | 0.13 |

A dash in the Tables indicates that no measurement was made. An asterisk indicates that the sterilisation step was omitted. The samples are identified by the weight percentage of fibre present in the product (5%, 6%, 7%), the fibre staple length (15mm, 50mm), and the liquor retention of the fibre (16g/g, 23 g/g, 27g/g, 31 g/g).

A sample of an alginate fibrous paste prepared according to US-A-5,482,932 exhibited a moisture donation value of 0.16 g/g. Its absorption capacities before and after sterilisation were 0.70 and 0.40 g/g respectively, and its stiffness before and after sterilisation was 548 and 233 cN respectively. These results indicate that sterilisation had marked adverse effects on the properties of the prior art paste.

### Example 2

Carboxymethylated lyocell fibre as used in Example 1 (10 mm staple) was added with stirring to sufficient propylene glycol or propylene glycol mixed with saline solution to permit fibre dispersion without appreciable swelling. Stirring was continued until the fibre was thoroughly wetted. Saline solution was then added whilst stirring was continued until the desired composition had been attained. The products contained 6% fibre and exhibited 27 g/g liquor retention. Measured properties of the products are given in Table 4:

**Table 4**

| | | | |
|---|---|---|---|
| Propylene glycol % w/w | 0 | 10 | 20 |
| Absorption Capacity g/g | - | 0.70 | - |
| Stiffness cN | - | 360 | - |
| Moisture Donation g/g | 0.11 | 0.07 | 0.02 |
| Moisture Affinity g/g | 0.04 | 0.07 | 0.10 |

Moisture affinity was measured in the same manner as moisture donation, except that freshly prepared 1% agar solution was used in place of gelatin solution. Agar solution was prepared as follows. To 1.00 ± 0.01 g agar powder (Oxoid technical Grade No. 3) in a sealable container is added sufficient sodium chloride and calcium chloride solution BP to produce a total weight of 100 ± 0.02 g. The resulting mixture is held at 121°C in an autoclave for 20 minutes and allowed to cool to approximately 60°C prior to use.

### Example 3

Water-swellable carboxymethyl cellulose powder (SW3009-X210, Trade Mark of Akzo Nobel NV) was added with stirring to propylene glycol. To this slurry was added with stirring carboxymethylated lyocell fibre as used in Example 1 (10 mm staple) . Stirring was continued until the fibre was thoroughly wetted. Saline solution was then added whilst stirring was continued until the desired composition had been attained. The product contained 5% of the fibre, 1% of the powder, and 20% propylene glycol. The product exhibited stiffness 419 cN, moisture donation 0.03 g/g and moisture affinity 0.22 g/g.

### Example 4

Example 3 was repeated, except that a water-soluble carboxymethyl cellulose powder was used in place of the water-swellable carboxymethyl cellulose powder. The product exhibited stiffness 369 cN, moisture donation 0.02 g/g and moisture affinity 0.23 g/g.

## Claims

1. A method for the manufacture of a material suitable for use as a wound dressing, **characterised in that** from 2 to 10 parts by weight of polymer solids, said polymer solids consisting of at least 50 percent by weight of water-swellable cellulose ether fibre and no more than 50 percent by weight of a powder which is a water-swellable or water-soluble cellulose ether or other water-swellable or water-soluble polymer, and correspondingly from 98 to 90 parts by weight of an aqueous liquor, are mixed under low-shear conditions, thereby forming said material in the form of a viscous paste exhibiting visible fibrous structure.

2. A method according to claim 1, **characterised in that** the polymer solids consist only of cellulose ether fibre.

3. A method according to either claim 1 or claim 2, further **characterised in that** the cellulose ether fibre is carboxymethyl cellulose fibre.

4. A method according to any one of the preceding claims, further **characterised in that** the degree of substitution of the cellulose ether is in the range from 0.1 to 1.0, preferably from 0.2 to 0.5.

5. A method according to any one of the preceding claims, further **characterised in that** the aqueous liquor comprises from 0.1 to 5.0 percent by weight sodium chloride.

6. A method according to any one of the preceding claims, further **characterised in that** the aqueous liquor comprises from 5 to 30 percent by weight propylene glycol.

7. A wound dressing which comprises a viscous paste exhibiting visible fibrous structure, **characterised in that** the paste consists of from 2 to 10 percent by weight of polymer, at least 50 percent by weight of said polymer being a water-swellable polymer and the balance (if any) of said polymer being a water-soluble polymer, and at least 50 percent by weight, of said polymer being a cellulose ether, and correspondingly of from 98 to 90 percent by weight of an aqueous liquor.

8. A wound dressing according to claim 7, **characterised in that** the polymer consists only of cellulose ether.

## Patentansprüche

1. Verfahren zur Herstellung eines als Wundverband geeigneten Materials, **dadurch gekennzeichnet, daß** man 2 bis 10 Gewichtsteile eines polymeren Feststoffes, bestehend aus mindestens 50 Gewichtsprozent einer wasserquellbaren Celluloseetherfaser und höchstens 50 Gewichtsprozent eines Pulvers, bei dem es sich um einen wasserquellbaren oder wasserlöslichen Celluloseether oder um ein anderes wasserquellbares oder wasserlösliches Polymer handelt, und entsprechend 98 bis 90 Gewichtsteile einer wäßrigen Flüssigkeit unter schwachscherenden Bedingungen mischt, wobei man das Material in Form einer viskosen Paste mit erkennbar faseriger Struktur erhält.

2. Verfahren nach Anspruch 1, **dadurch gekennzeichnet, daß** der polymere Feststoff ausschließlich aus Celluloseetherfaser besteht.

3. Verfahren nach einem der Ansprüche 1 oder 2, weiterhin **dadurch gekennzeichnet, daß** man als Celluloseetherfaser Carboxymethylcellulosefaser einsetzt.

4. Verfahren nach einem der vorhergehenden Ansprüche, weiterhin **dadurch gekennzeichnet, daß** der Substitutionsgrad des Celluloseethers im Bereich von 0,1 bis 1, 0 und vorzugsweise im Bereich von 0,2 bis 0,5 liegt.

5. Verfahren nach einem der vorhergehenden Ansprüche, weiterhin **dadurch gekennzeichnet, daß** die wäßrige Flüssigkeit 0,1 bis 5,0 Gewichtsprozent Natriumchlorid enthält.

6. Verfahren nach einem der vorhergehenden Ansprüche, weiterhin **dadurch gekennzeichnet, daß** die wäßrige Flüssigkeit 5 bis 30 Gewichtsprozent Propylenglykol enthält.

7. Wundverband aus einer viskosen Paste mit erkennbar faseriger Struktur, **dadurch gekennzeichnet, daß** die Paste aus 2 bis 10 Gewichtsprozent Polymer, wobei es sich bei mindestens 50 Gewichtsprozent des Polymers um ein wasserquellbares Polymer und bei dem etwaigen Rest um ein wasserlösliches Polymer handelt und bei mindestens 50 Gewichtsprozent des Polymers um einen Celluloseether handelt, und entsprechend aus 98 bis 90 Gewichtsprozent einer wäßrigen Flüssigkeit besteht.

8. Wundverband nach Anspruch 7, **dadurch gekennzeichnet, daß** das Polymer ausschließlich aus Celluloseether besteht.

## Revendications

1. Procédé de fabrication d'un matériau approprié pour une utilisation comme pansement pour plaies, **caractérisé en ce que** l'on mélange de 2 à 10 parties en poids de matières sèches de polymère, lesdites matières sèches de polymère étant constituées d'au moins 50 pour cent en poids de fibre d'éther de cellulose gonflable dans l'eau et de pas plus de 50 pour cent en poids d'une poudre qui est un éther de cellulose gonflable dans l'eau ou soluble dans l'eau ou un autre polymère gonflable dans l'eau ou soluble dans l'eau, et, de façon correspondante, de 98 à 90 parties en poids d'une liqueur aqueuse, dans des conditions de faible cisaillement, pour mettre ledit matériau sous la forme d'une pâte visqueuse présentant une structure fibreuse visible.

2. Procédé selon la revendication 1, **caractérisé en ce que** les matières sèches de polymère ne se composent que de fibre d'éther de cellulose.

3. Procédé selon la revendication 1 ou la revendication 2, **caractérisé en outre en ce que** la fibre d'éther de cellulose est de la fibre de carboxyméthylcellulose.

4. Procédé selon l'une quelconque des revendications précédentes, **caractérisé en outre en ce que** le degré de substitution de l'éther de cellulose est dans la gamme de 0,1 à 1,0, de préférence de 0,2 à 0,5.

5. Procédé selon l'une quelconque des revendications précédentes, **caractérisé en outre en ce que** la liqueur aqueuse comprend de 0,1 à 5,0 pour cent en poids de chlorure de sodium.

6. Procédé selon l'une quelconque des revendications précédentes, **caractérisé en outre en ce que** la liqueur aqueuse comprend de 5 à 30 pour cent en poids de propylèneglycol.

7. Pansement pour plaies qui comprend une pâte visqueuse présentant une structure fibreuse visible, **caractérisé en ce que** la pâte se compose de 2 à 10 pour cent en poids de polymère, au moins 50 pour cent en poids dudit polymère étant un polymère gonflable dans l'eau et le reste (éventuellement) dudit polymère étant un polymère soluble dans l'eau, et au moins 50 pour cent en poids dudit polymère étant un éther de cellulose, et, de façon correspondante, de 98 à 90 pour cent en poids d'une liqueur aqueuse.

8. Pansement pour plaies selon la revendication 7, **caractérisé en ce que** le polymère se compose seulement d'éther de cellulose.
